# EUROPEAN PATENT APPLICATION

(11) **EP 4 792 739 A2**
(43) Date of publication of application: **19.08.2026**
(21) Application number: 26174086.4
(22) Date of filing: 04.05.2021
(51) Int. Cl.: A61B 5/021

(54) **VASCULAR FLOW AND PRESSURE MODULATOR**

(30) Priority: 04.05.2020 US 202063019628 P
(62) Document number(s) of the earlier application(s) in accordance with Art. 76 EPC: 21732611.5 / 4 146 093
(71) Applicant: Vahaticor, Inc., Los Gatos, CA 95032 (US)
(72) Inventor: BERRADA-SOUNNI, Marwan, Los Gatos, 95032 (US); VAN BLADEL, Kevin H, Livermore, 94550 (US)
(74) Representative: Marks & Clerk LLP

(57) **Abstract**

Disclosed are vascular flow modulators generally comprised of an expandable scaffold with built-in adjustability to modulate hemodynamic output inside a vessel, for instance, the coronary sinus. Methods of placing and adjusting disclosed flow modulators are also disclosed.

## Description

### RELATED APPLICATIONS

The present application claims priority to U.S. Provisional Application No. 63/019,628, filed May 4, 2020, and entitled "Vascular Flow and Pressure Modulator", which is incorporated herein by reference in its entirety.

### FIELD OF THE DISCLOSURE

The present disclosure relates to vascular interventional devices and methods, and more specifically, to vascular flow and pressure modulators and related systems and methods.

### BACKGROUND

A flow/pressure modulator is a vascular restrictor used by physicians, typically interventional cardiologists, to modulate hemodynamic flows and pressures to induce an artificial physiological effect beneficial to the patient that the current physiological system cannot achieve. The vascular restrictor is variable and of a specific diameter personalized to the patient's needs at the time of implantation. The application of such a device is broad to all types of vessels (arterial and venous), and more particularly for reducing flow and augmenting pressure in the coronary sinus to benefit refractory angina patients.

A coronary sinus flow/pressure modulator is a device to aid in the management of patients with angina refractory to optimal medical therapy and not amenable to further revascularization. Conventionally, the device is a controllable flow-limiting scaffold providing a hemodynamic restructure within the coronary sinus lumen. The intention is to increase back pressure within the coronary sinus to drive higher perfusion to the distal coronary bed and redistribute trans-myocardial blood flow. Currently available devices are typically configured as a generally hourglass-shaped porous scaffold that endothelializes into the coronary sinus wall to create a reduced diameter orifice. However, until the scaffold is entirely or close to entirely endothelialized, potential therapeutic effect is not fully realized. Additionally, the time to the determination of the effectiveness of treatment can exceed thirty days and generally will happen at different rates for different patients depending on individual patient response. As a result, therapeutic results with current devices are unpredictable and at best well-delayed after the initial placement procedure. Furthermore, the ability to modulate the hemodynamic restriction of coronary sinus blood flow is a feature lacking in current clinically available devices, which function on a "one-size fits all" premise.

### SUMMARY OF THE DISCLOSURE

In some disclosed embodiments, a vascular flow modulator, comprises an expandable tubular structure having first and second ends with a central portion therebetween. Means forming an adjustable flow restriction through said central portion are provided and a thrombosis-resistant surface is disposed within the flow restriction. In certain embodiments the means forming an adjustable flow restriction comprises means forming an adjustable reduced diameter section of the central portion. In further embodiments, the means forming an adjustable reduced diameter section comprise means for adjusting the reduced diameter portion after the flow modulator is deployed within a vascular lumen of a patient.

In another aspect of the present disclosure, vascular flow modulator kit are described including an open cell expandable tubular structure having first and second ends with a narrowed central portion therebetween. A first sleeve material having at least an inner thrombosis-resistant surface and formable into a plurality of first sleeves of different lengths and inner diameters is provided. The first sleeves when placed around the narrowed central portion of the expandable tubular structure constrain the narrowed central portion at a selected inner diameter and length with an inward facing thrombosis resistant surface. The kit further may include a second sleeve material having at least an outer tissue in-growth promoting surface and formable into a plurality of second sleeves of different lengths and diameters. The second sleeves are configured to surround at least the first sleeve to provide an outward facing tissue in-growth promoting surface. In some embodiments the first and second sleeve materials may be joined as opposites sides of a flat-self adhering sheet that can be formed into a sleeve or as a bi-layer, self-adhering material.

In a further aspect of the present disclosure, methods of modulating vascular flow are described. Embodiments of the described methods comprise preparing a vascular flow modulator for placement in a patient, delivering the vascular flow modulator through the patient's vasculature to a treatment site, placing the vascular flow modulator within a vascular lumen at the treatment site, and adjusting a narrowed flow reducing portion of the vascular flow modulator based on specific patient clinical need. In some embodiments, the adjusting of the narrowed flow reducing portion comprises configuring the vascular flow modulator during the preparing step. In other embodiments, the adjusting of the narrowed flow reducing portion comprises changing an internal diameter of the narrowed flow reducing portion after placing the vascular flow modulator in the vascular lumen.

### BRIEF DESCRIPTION OF THE DRAWINGS

To illustrate the disclosure, the drawings show aspects of one or more embodiments of the disclosure. However, it should be understood that the present disclosure is not limited to the precise arrangements and instrumentalities shown in the drawings, wherein:
- FIG. 1: is a schematic side and end view of the first embodiment of a vascular flow modulator according to the present disclosure.
- FIG. 2: is a schematic side view of an alternative embodiments of a vascular modulator according to the present disclosure.
- FIG. 2A: is a schematic cross-sectional view through line A-A of FIGS. 2 and 2B.
- FIG. 2B: is a schematic cross-sectional view through line B-B of FIGS. 2 and 2B.
- FIG. 2C: is a schematic cross-sectional view through line A-A of FIGS. 2, 2B and 2D showing a further alternative inner configuration of the embodiments of FIG. 2.
- FIG. 2D: is a schematic cross-sectional view through line B-B of FIGS. 2, 2A and 2C showing a further alternative inner configuration of the embodiments of FIG. 2.
- FIG. 3: is a schematic side and end view of another alternative embodiment of a vascular flow modulator according to the present disclosure, with the end view depicting a partially collapsed or partially expanded state of the device.
- FIG. 4: is a schematic side view of a further alternative embodiment of a vascular flow modulator according to the present disclosure.
- FIG. 5: is a schematic side view of yet another alternative embodiment of a vascular flow modulator according to the present disclosure.
- FIG. 6: is a schematic side view of a further alternative embodiment of a vascular flow modulator according to the present disclosure.
- FIG. 6A: is a schematic side view of a vascular flow modulator as in FIG. 6 communicating with a balloon fluid reservoir.
- FIG. 7: is a schematic side view of an additional alternative embodiment of a vascular flow modulator according to the present disclosure.
- FIG. 8: is a schematic side view of a further alternative embodiment of a vascular flow modulator according to the present disclosure.
- FIG. 9: is a schematic side view of a further alternative embodiment of a vascular flow modulator according to the present disclosure.
- FIG. 10: is a schematic side view of another alternative embodiment of a vascular flow modulator according to the present disclosure.
- FIG. 11: is a schematic side view of another alternative embodiment of a vascular flow modulator according to the present disclosure.
- FIG. 12: is a schematic side view of another alternative embodiment of a vascular flow modulator according to the present disclosure.
- FIG. 13: is a schematic side view of another alternative embodiment of a vascular flow modulator according to the present disclosure.
- FIG. 13A: is a partial cut-away side view of the embodiment shown in FIG. 13.
- FIG. 13B: is another partial cut-away side view of the embodiment shown in FIG. 13 in an alternative configuration.

### DETAILED DESCRIPTION

Devices described in the present disclosure generally comprise of an expandable scaffold with built-in adjustability to modulate hemodynamic output inside a vessel, for instance, the coronary sinus. Flow modulation structures disclosed herein generally comprise three functional aspects: anchoring structure configured to fix the device in a vascular lumen and avoid device embolization, a flexible structure configured to create a flow/pressure reduction without excessively restricting flow, and an adjustment mechanism to provide for modulation of the flow/pressure alteration adapted to changing patient requirements. Conical or straight configurations are used as different structural platforms in various disclosed embodiments. Conical structures perform both the anchoring of the device in addition to the flow diversion, and the following settings have a straight section apposing the lumen to anchor the device to the anatomy and inside such structures a flow-diverting method to create a controllable pressure drop between the inlet and the outlet of the device. Straight configurations could be formed either with a restriction invaginated into the structure of a two-part structure whereby the outside structure is self-expandable acting as an anchor. In contrast, the inner part may be formed from a fixed structure (balloon-type material with a flow-diverting shape and a restriction or a stainless-steel material expandable with a balloon). Disclosed embodiments are thus configured to provide a therapy customized to the patient's hemodynamic environment, which are useful in a short timeframe and/or adjustable in-situ, adapted to the patient's hemodynamic anatomy based on a clinically relevant pressure drop, and optionally with sensors to allow dynamic adjustment and/or a patient's longitudinal follow-up.

Embodiments disclosed herein address concerns identified with existing coronary sinus flow modulation solutions through the deployment of a personalized restriction to maintain a specific pressure gradient. Such gradient can also be attained through the creation of hydraulic resistance, forcing blood to flow through added tortuosity, artificially created using small polytetrafluoroethylene (PTFE) (or equivalent) tubes twisted together to add a hydraulic head without providing a flow restriction.

Features for monitoring and maintaining such restriction also may be included, as described herein. Monitoring is through sensors, either passive or active (e.g., requiring battery). Depending on the hemodynamic feedback, the restriction may be adjusted either automatically in-situ through an external stimulus or internally through a simple procedure (e.g., angioplasty balloon, or inflation of a sac). Embodiments of the disclosed coronary sinus flow modulators present a self-expandable structure capable of conforming to the anatomy. The coronary sinus anatomy is not only conical, but also includes a curvature, and anatomical variability exists among patients. Therefore, the ability to have different sizes with a variable shape that could be placed more distally relative to current devices may be further advantageous.

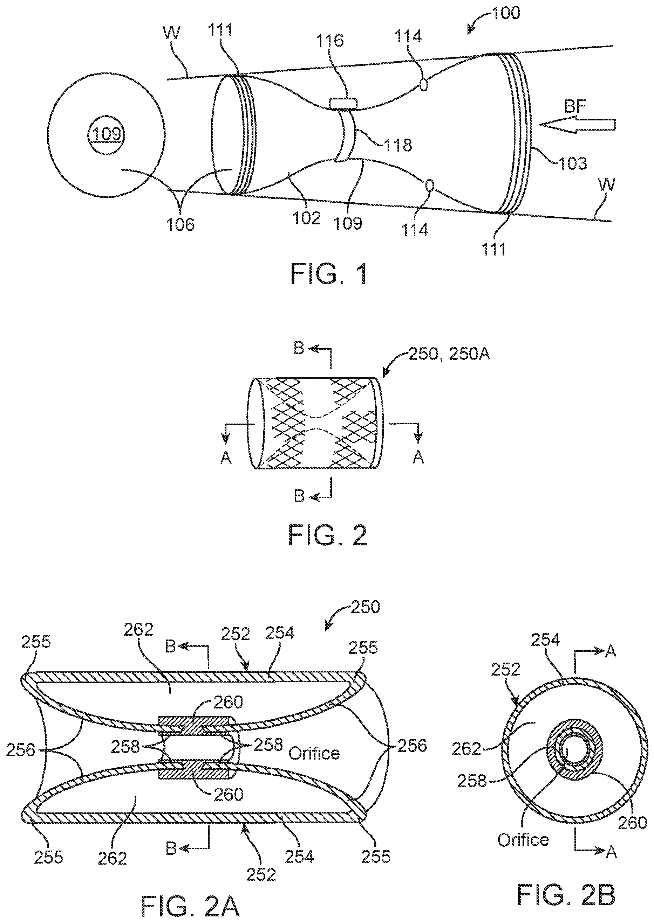

In one embodiment, as shown in FIG. 1, coronary sinus flow modulator 100 comprises molded body 102 made from a biocompatible material such as polyurethane, Grilamid^{®}, Nylon 12, and Nylon 6. When implanted in the coronary sinus, blood flow (BF) is in the direction indicated by arrow BF, entering distal end opening 103 and exiting proximal end opening 106. The general hourglass shape of body 102 with central narrowed orifice region 109 may be formed by molding the body material in that shape. Other shapes, as shown herein, may also be employed. Nitinol or other resilient material rings 111 are provided around the outside of distal opening 103 and proximal opening 106 to allow for collapse and expansion of body 102 for transport through the vasculature to and expansion at the deployment site to match the patient anatomy, including for patients whose coronary sinus reduces in diameter from the opening inward. Resilient rings 111 may be formed, for example, from Nitinol wire or braid and may also serve as anchors for body 102 and may further include anchor features such as barbs or coils, etc. Multiple different sizes may be provided to accommodate different patient sizes and clinical situations.

Fenestrations or openings 114 in body 102 allow for entry of blood when the device is first deployed in the coronary sinus. Blood will fill the void area between the vessel wall and narrowed region 109 and coagulate therein in order to provide an immediate or nearly immediate functioning, non-collapsing orifice upon deployment, which does not require time to endothelialize. Further, electronic package 116 may be provided on an outer surface of body 102 in narrowed region 109. Electronic package 116 may include monitoring devices such as pressure or flow sensors that wirelessly communicate with a detection system outside the patient's body to provide information on the patient's hemodynamic anatomy. Electronic package 116 may be optionally included with any of the coronary sinus flow modulator embodiments disclosed herein.

In a further alternative, electronics package 116 may be optionally provided to control the diameter of adjustable ring 118 based on pressure measurements or other patient flow metrics. For example, adjustable ring 118 may interface with a micro-motor driven gear mechanism within electronic package 116 so as to dynamically set the diameter of narrowed section 109 larger or smaller in response to pressure or flow changes as measured by sensors, such as a capacitive pressure sensor, included in the electronic package. An example of an interface between the micro-motor and adjustable ring is a worm or spur gear drive engaging with a flexible gear rack, such as a "zip-tie" surface, on adjustable ring 118.

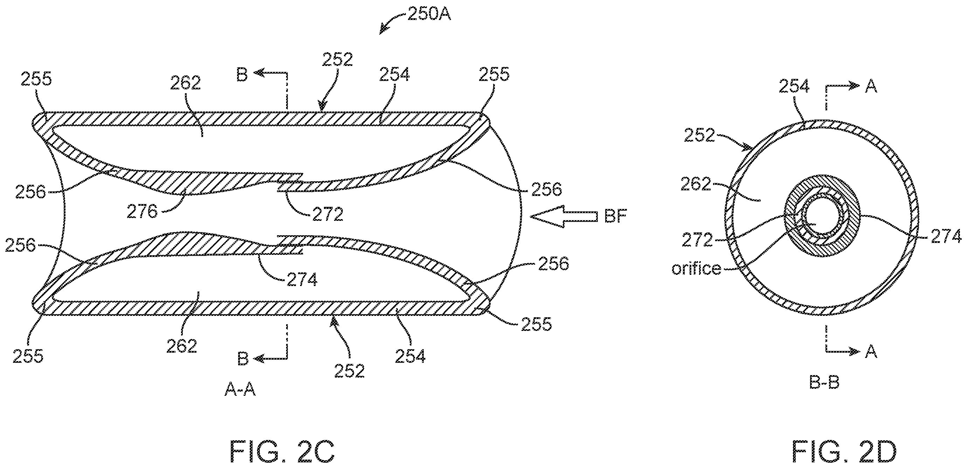

FIGS. 2, 2A and 2B illustrate another embodiment in which flow and pressure modulator 250 is formed by invaginated mesh tube 252 with a straight outer wall 254, bends 255 where inwardly directed portions 256 of mesh tube 252 are turned back inward and connected at ends 258 by an annular crimp or collar 260 forming a reduced diameter flow passage. Void areas 262 are defined between straight outer wall and inwardly directed portions 256. The shape and size of modulator 250 may be varied by varying the amount of distance set between opposed ends 258 within annular collar 260. The diameter of the reduced diameter flow passage may be adjusted using annular collars with different inside diameters. The mesh of tube 252 may be covered with a flexible material such as silicone or polyurethane. Annular collar 260 may be made of a thrombosis-resistant material or have a thrombosis-resistant coating on its inner surface.

FIGS. 2C and 2D illustrate an alternative inner configuration for the flow modulator 250, designated as 250A. The outward appearance of flow modulator 250A is the same as flow modulator 250, as shown in FIG. 2, and in many respects it is similar to flow modulator 250, such that only the differences are described in this paragraph. Instead of using a crimp collar to join opposed ends 272 and 274. On one side, proximally positioned with respect to end 274, inwardly directed portion 256 includes an raised annular orifice adjustment ridge 276. Thus, the inner diameter of the orifice can be adjusted based on how far end 272 is inserted into end 274. Flow modulator 250 may be provided pre-invaginated with end 272 received in end 274 at the widest orifice or it may be provided as a straight tube and invaginated by the physician prior to placement. In either case, the physician may set a desired orifice inner diameter prior to placement by adjusting the distance end 272 is received in end 274. The orifice diameter also may be adjusted *in vivo* by similar manipulations.

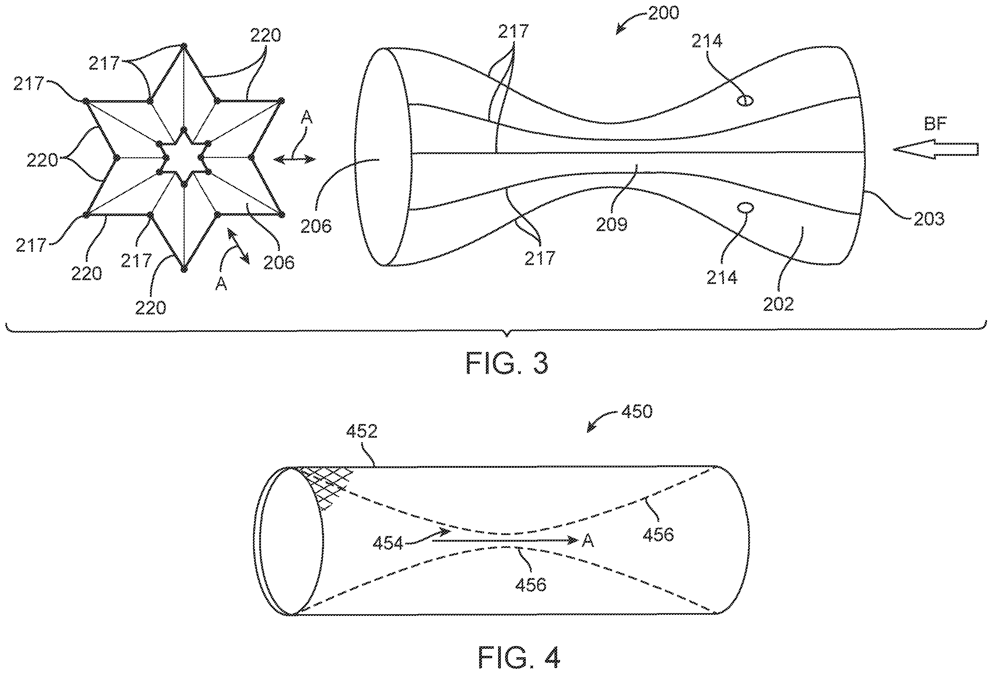

In another embodiment, as shown in FIG. 3, coronary sinus flow modulator 200 comprises body 202 defining open distal end 203 and open proximal end 206 with narrowed region 209 in between forming an orifice. This embodiment also provides a fixed diameter orifice. Blood flow is in the direction of arrow BF, entering through the distal end opening 203 and exiting from proximal end opening 206. Openings or fenestrations 214 in body 202 allow entry of blood into the void areas defined between the coronary sinus wall and narrowed region 209, which will coagulate and support narrowed region 209 and the orifice formed thereby relatively quickly upon deployment of flow modulator 200 in the coronary sinus.

The configuration of flow modulator 200 allows it to collapse in a star-like configuration, as shown in the end view of FIG. 3, to provide a low-profile package for delivery through the vasculature to the coronary sinus. Plural nitinol longitudinal frames 217 extend along the length of body 202, joined by plural transverse nitinol frame members 220. The nitinol frame members 217 and 220 are covered with a sheet material used for angioplasty balloons, such as Polyethylene terephthalate (PET), expanded Polytetrafluoroethylene (ePTFE), nylon or other thrombosis-resistant materials. The sheet may be pre-formed or molded into the hourglass shape shown so that when fully expanded, the desired shape with narrowed portion 209 is maintained. Arrows A in the end view of FIG. 3 indicate the direction of movement of alternating longitudinal frame members during collapse or expansion of flow modulator 200. Flow modulator 200 also may utilize electronics package 116, as shown in FIG. 1 and may be provided with an adjustable reduced diameter portion using means disclosed in other embodiments herein.

In another alternative embodiment, as illustrated in FIG. 4, vascular flow and pressure modulator 450 includes an, at least, substantially straight outer mesh tube 452 with an inner reducing orifice 454 formed in a necked annular inner member 456, which may be made of PET, ePTFE, Nylon-6, Nylon-12 or other materials used, for example, for angioplasty balloons. Inner member 456 is flexible, thus once attached inside mesh tube 452, changes in the length of tube 452 will change the diameter of orifice 454, *i.e.,* elongating tube 452 will open the orifice, whereas mesh tube 452 will narrow the orifice. Anchor members may be provided around one or both ends of outer mesh tube 452 as elsewhere described in this disclosure. Mesh tube 452 may be left as an open mesh to encourage tissue in-growth or may have a coating such as polyurethane PET, or ePTFE as also elsewhere described herein. Arrow (A) indicates a blood flow direction. In another variation, both outer mesh tube 452 and annular inner member 456 are formed as a continuous mesh structure, but with the inner member coated by a flexible, thrombosis-resistant material as listed elsewhere herein to provide the reduced diameter flow path.

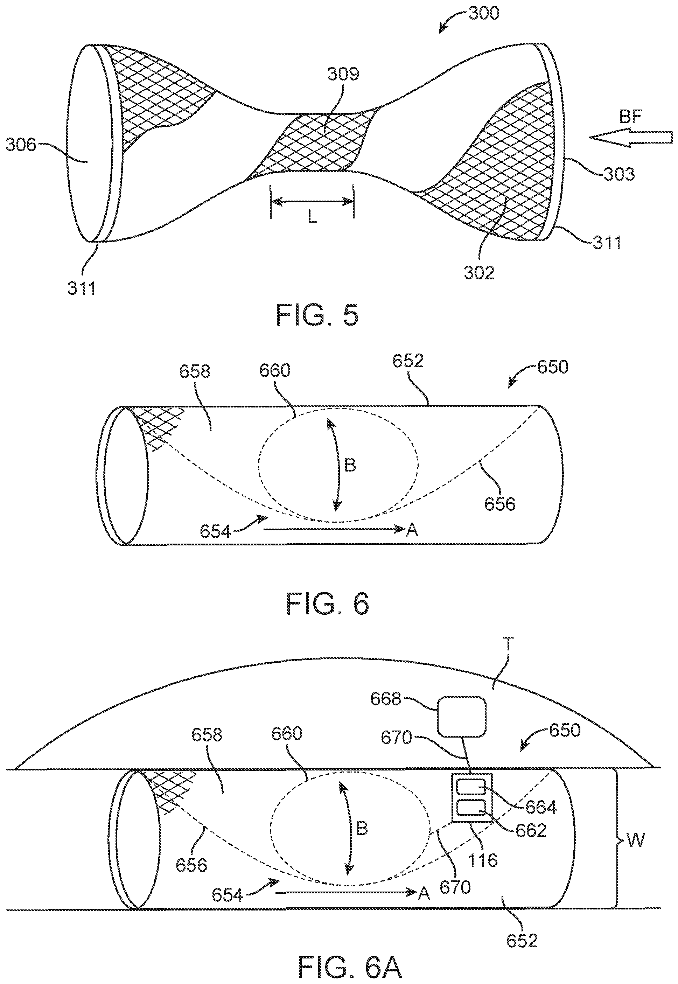

In a further alternative embodiment, as shown in FIG. 5, vascular flow modulator 300 includes a variable or adjustable diameter orifice. In this embodiment, flow modulator body 302 defines distal open end 303 for receiving blood flow as indicated by arrow BF and a proximal open end 306 from which blood flow exits. Body 302 is formed of a wire braid structure defining cells coated with a polyurethane coating, PET, ePTFE or similar thrombosis-resistant material. The configuration of the wire braid structure allows the diameter of the orifice defined by narrowed region 309 to be adjusted when the length L of narrowed region 309 is changed. In one configuration, increasing the length L causes the diameter of the orifice to proportionally increase, whereas shortening the length L has the opposite effect of decreasing the diameter of the orifice proportionally. In other embodiments, the wire mesh is constructed such that shortening length L increases the orifice inside diameter. Anchor wire or braid 311 may be provided around each open end.

An alternative braided structure for body 302 (and other disclosed tubular bodies) includes nitinol (shape-memory alloy) wire and/or bio-absorbable filament embedded in the structure to initially promote endothelization and then be absorbed. The braid structure could be coated with PET, Polydioxanone (PDS), Polyethylene glycol/Polylactic acid (PEG/PLA) or Polyglycolic acid/Polyhydroxyalkanoate (PGA/PHA) a functionalized coating design to achieve the purpose of either promoting or preventing endothelization depending on the desired location (*e.g.,* no endothelization around narrowed region 309). Options for braided structure include compact (*e.g.,* high pick-per-inch) or open-cell filled with stretchable material such as polyurethane. Braid 311 could also be a substrate for electrospinning material to be deposited to create a restriction.

In another alternative embodiment, as illustrated in FIG. 6, vascular flow and pressure modulator 650 includes an at least substantially straight outer mesh tube 652 with an inner reducing orifice 654 formed in a necked asymmetric inner member 656, which may be made of PET, ePTFE, Nylon- 6, Nylon-12 or other materials used, for example, for angioplasty balloons. Modulator 650 is in many respects similar to modulator 450, except for the asymmetric arrangement of inner member 656. Inner member 656 is flexible, thus once attached inside mesh tube 652, changes in the length of tube 652 may change the diameter of orifice 654. Alternatively, injection of a gel or other similar substance into void 658, or a balloon 660 located therein, may be used to adjust the size of orifice 654. Anchor members may be provided around one or both ends of outer mesh tube 652 as elsewhere described in this disclosure. Mesh tube 652 may be left as an open mesh to encourage tissue in-growth or may have a coating such as polyurethane as also elsewhere described herein. Arrow (A) indicates a blood flow direction, and arrow (B) indicates adjustment direction.

In one further alternative embodiment, as shown in FIG. 6A, the electronics package 116 (*e.g.,* also FIG. 1) may include a micropump 662 and controls 664 to provide and control fluid communication between balloon 660 and fluid reservoir 668, which may be implanted outside the vessel wall (W) in surrounding tissue (T) and communicate with balloon 660 through fluid channel 670, via micropump 662. Micropump 662 could then dynamically control balloon size in response to inputs from pressure and flow sensors as elsewhere described therein. Such an arrangement with a balloon 660 may be employed in symmetric embodiments like modulator 450 as well as asymmetric embodiments like modulator 650.

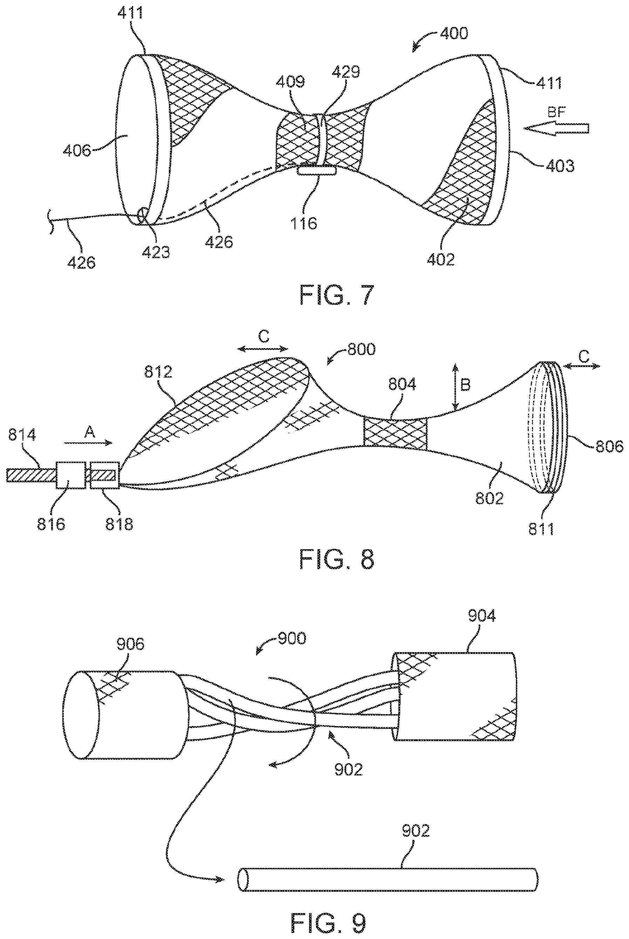

In another alternative embodiment, as shown in FIG. 7, vascular flow modulator 400 includes body 402 formed of a wire braid or similar braided material with polyurethane coating. In another alternative, a frame structure could be laser cut from a nitinol tube rather than provided as a braided wire structure. Blood flow direction is entering through the open distal end 403 and exiting open proximal end 406, in the direction of arrow BF. Narrowed region 409 provides a reduced, variable diameter orifice between the two ends. Anchor wire or braid 411 is provided around each open end and also may serve to stiffen and maintain the openings.

In flow modulator 400, the diameter of the reduced diameter orifice is controlled by adjustable ring 429 surrounding narrowed region 409. In one embodiment, the diameter of adjustable ring 429 may be changed by pulling or releasing tether 426, which can be secured at a specific position by stop nut or slide stop 423. In a further alternative, electronics package 116 may be optionally provided to control the diameter of adjustable ring 429 based on pressure measurements. For example, adjustable ring 429 may be initially set at a minimum diameter with a bias towards open that is limited by tether 426. Movement within a capacitive pressure sensor in electronics package 116 may release tether 426 in response to pressure changes that would then permit adjustable ring 429 to bias more open to increase flow and decrease backpressure.

In a another alternative embodiment illustrated in FIG. 8, vascular flow and pressure modulator 800 includes a mesh body 802 as previously described, which may have a coating to close openings between the wire mesh. Coating materials may include, for example, silicone, PET, ePTFE or polyurethane, with or without additional thrombosisresistant coatings or treatments. Mesh body 802 is formed with narrowed central region 804, which forms an internal flow-restricting orifice and widened outlet end 806. At least one tissue anchor 811 is provided around outlet end 806. The inlet end 812 is also enlarged and may be formed at an oblique angle to the direction of blood flow, as indicated by the arrow (A). The inner diameter of the orifice in narrowed central region 804 may be controlled or varied by moving the ends 806, 812 relative to each other as indicated by arrows (C) to cause narrowed central region 804 to expand or contract as indicated by the arrow (B).

Adjustment of the diameter of narrowed central region 804, as described above, may be accomplished using retrieval cable 814. Modulator 800 is released from a delivery device into the coronary sinus with outlet end 806 first released. Outlet end 806 then expands so that anchors 811 engage the lumen wall. Once engaged, the length of modulator 800 may be adjusted by pushing or pulling retrieval cable 814 to push ends 806, 812 together, or pull them apart. Retrieval cable 814 also may be used to pull the entire device back into a guide catheter or other delivery sheath (not shown) for removal of the device or replacement in the desired location. Fittings 816 and 818 provide a threaded connection between retrieval cable 814 such that the retrieval cable may be unscrewed by rotation and removed when desired placement is confirmed. In a further alternative embodiment, retrieval cable 814 may be formed with inner and outer relative sliding coaxial members to control the opening or closing of a lasso member around inlet open end 812. Such a lasso arrangement may provide additional control and options for sizing and shaping modulator 800 in vivo after release from the delivery device.

FIG. 9 illustrates yet another embodiment in which a pressure gradient is attained through the creation of hydraulic resistance, i.e., by forcing blood to flow through added tortuosity in device 900 created by plural PTFE (or equivalent) tubes 902 twisted together. Such an arrangement may add a hydraulic head without a substantial flow restriction. The degree of tortuosity may be varied, and thus the amount of added hydraulic head varied by rotating mesh inlet and outlet collars 904, 906 relative to one another.

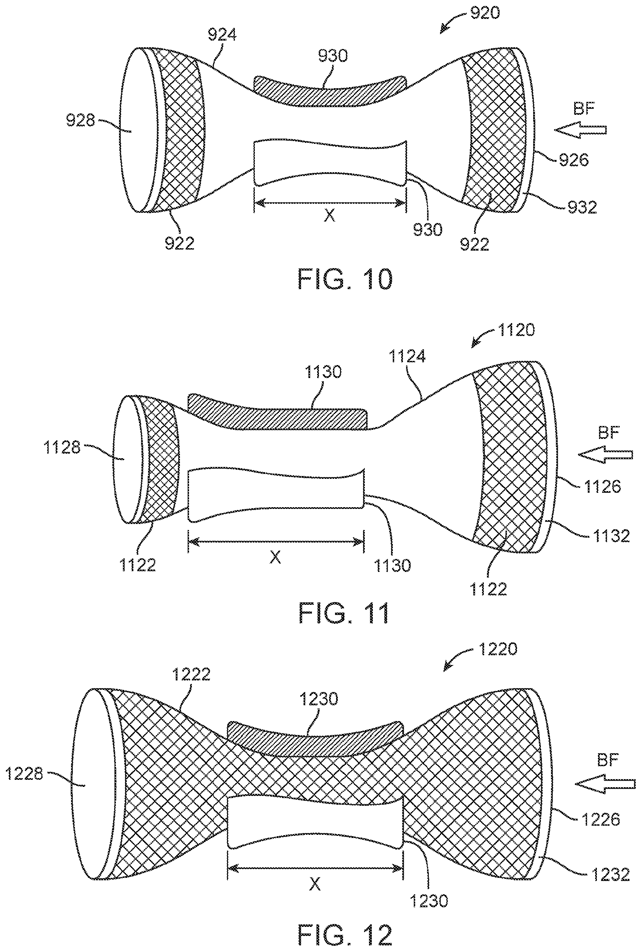

In a further alternative embodiment, flow modulator 920, shown in FIG. 10, is formed substantially as flow modulator 100 (FIG. 1), but with open cell/mesh end regions 922 at both ends 926, 928, and a central, closed-cell, flow-facilitating central region 924 wherein the reduced diameter orifice is formed. An adjustable length sleeve 930 surrounds the reduced diameter orifice portion of flow modulator 920. In some embodiments, sleeve 930 is provided as a non-resilient member with a fixed inner diameter so that the self-expanding body of flow modulator 920 conforms to the shape of sleeve 930 thereby setting the diameter and length of the reduced diameter orifice portion. Multiple sleeves with different lengths and inner diameters may be provided to permit the physician to precisely craft the flow modulator to the specific patient requirements. Sleeves 930 also may be fixed length or provided as longer tubes that are cut to length at the time of implantation, thus offering further customization options. In a further alternative, the material for sleeve 930 may be provided as a flat sheet or strip of self-adhering fabric, such as double-sided hook-and-loop fastener fabric, such that the sleeve may be formed to any desired diameter and length just prior to placement based on the anatomical structure presented at the time of the procedure.

In some embodiments, particularly when the mesh structure is encapsulated in or coated with a thrombosis-resistant material to provide a smooth, closed-cell section to limit thrombosis within reduced diameter portion as in FIG. 10, sleeve 930 may be comprised of tissue in-growth promoting materials such as polyester or Dacron or a bioabsorbable material such as polydioxanone (PDS), polyethylene glycol/polylactic acid (PEG/PLA) or polyglycolic acid/polyhydroxyalkanoate (PGA/PHA). In this configuration, sleeve 930 would promote in-growth of tissue toward the orifice reduction but has an adjustable length X as may be determined by the physician. Adjustable ring structures 932 as previously described optionally surround each of ends 926, 928. Flow modulator 920 provides an immediate effect of flow/pressure reduction within the coronary sinus due to closed-cell central region, while tissue in-growth sleeve 930 provides advantages of tissue in-growth in the region of the reduced orifice that is typically associated with open-cell structures.

As mentioned above, some patients may have a coronary sinus that decreases in size, requiring the flow modulator to have two different diameters of expansion in order to be properly positioned within the vessel. FIG. 11 illustrates one alternative embodiment for addressing this requirement. As shown therein, flow modulator 1120 has open-cell/mesh-end regions 1122 at ends 1126, 1128, flow facilitating central region 1124 wherein the reduced diameter orifice is formed, and optional resilient end rings 1132, substantially the same as flow modulator 920 in FIG. 10. However, as shown in FIG. 11, sleeve 1130 is positionable longitudinally along central region 1124 to allow for adjustability of the size of ends 1126, 1128. When sleeve 1130 is placed in the center, the two ends of the flow modulator would expand to the same diameter. However, if the sleeve was closer to one end, as shown in FIG. 11, that end would be more restricted and the overall diameter would be smaller, whereas the other end of the flow modulator would be less restricted which would allow the self-expanding device to expand to a larger diameter. Combined with the easily sizable and positionable sleeves, such as sleeves 930, 1130 and further examples below, these features provide greater flexibility for addressing different clinical situations or patient anatomies.

In another alternative embodiment, as shown in FIG. 12, flow modulator 1220 is formed with a continuous open mesh body 1222, for example, in the manner of a balloon-expandable or self-expanding stent structure. Ends 1226, 1228 optionally may be provided with resilient ring structures 1232 as described for other embodiments. In this embodiment, because the body is entirely an open mesh, positionable/sizeable sleeve 1230 is formed of a thrombosis-resistant material to reduce or prevent occlusion from thrombosis of the reduced diameter section created by the restricted diameter of sleeve 1230. Examples of thrombosis-resistant materials include, but are not limited to ePTFE and poly(ethylene oxide) (PEO)-silane amphiphiles modified silicones.

In another alternative embodiment, positionable sleeves as described herein, for example sleeve 1230 in FIG. 12, may comprise a multi-layer construction. In such an alternative, the inner layer may be made of a thrombosis-resistant material or have an inner thrombosis-resistant coating as elsewhere described herein, while the outer layer is made of a tissue in-growth promoting material as also elsewhere described herein.

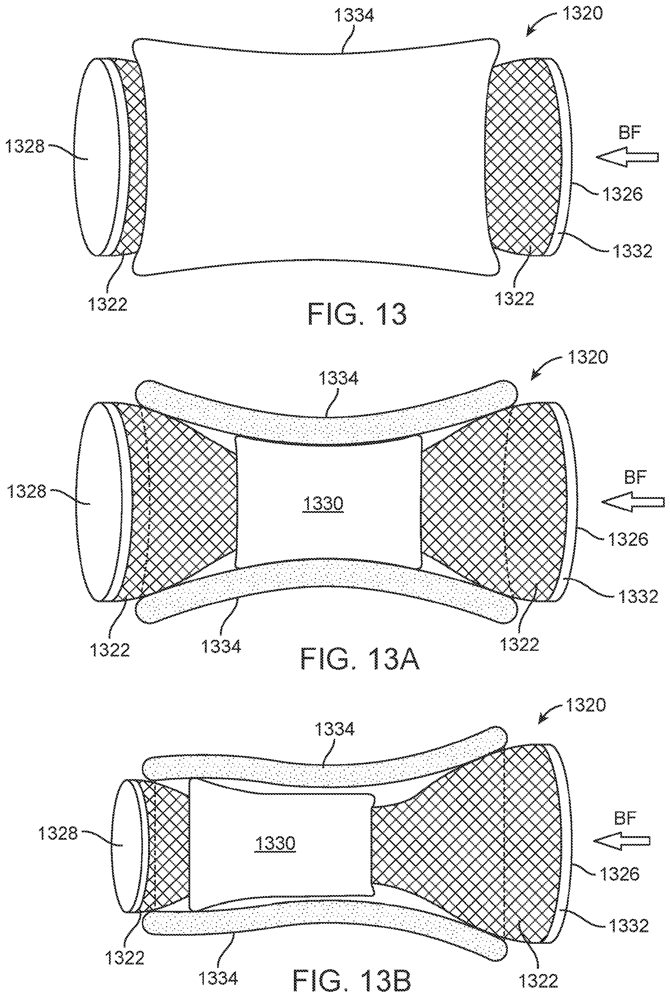

In a further alternative embodiment, as shown in FIGS. 13, 13A and 13B, flow modulator 1320 comprises inner mesh body 1322 with ends 1326, 1328 and optional end ring structures 1332, inner positionable sleeve 1330 defining a reduced diameter orifice section as described above, and outer tissue in-growth sleeve 1334. As with modulator 1220 (FIG. 12), inner positionable sleeve 1330 is preferably formed of a thrombosisresistant material to reduce or eliminate thrombosis in the narrowed orifice section. Outer tissue in-growth sleeve 1334 is preferably formed of a tissue in-growth promoting material as described above for sleeve 930 (FIG. 10). Outer sleeve 1334 also can provide a sealing effect for the outer ends of mesh structure 1322 so as to allow it to immediately direct blood flow through the reduced diameter portion upon placement for faster clinical effect.

With a structure such as shown in FIGS. 13 and 13A, many different configurations can be created with flow modulator 1320 to match a variety of patient anatomies and clinical conditions. Each of sleeves 1330 and 1334 may be provided as self-adhering flat sheets to be formed into sleeves of appropriate inner diameter before placement or as pre-formed tubes that can be cut to length before placement. By setting the diameter and longitudinal placement of inner sleeve 1330, configurations customized to the patient's needs as shown in FIG. 13B can be created at the time of placement. In a further alternative, by providing the material for sleeves 1330 and 1334 as a bi-layer, self-adhering material, with one surface being thrombosisresistant and the other surface being tissue in-growth promoting, a single sheet of flat material can be used and sized as needed for both sleeves. Thus, providing these components in a system or kit-like form can substantially reduce the number of pre-sized components as may be needed with prior systems to provide the same flexibility in meeting specific clinical situations or patient anatomies.

As will be appreciated by persons of ordinary skill based on the teachings contained herein, vascular flow modulators as disclosed herein may be formed as self-expanding structures or balloon expandable structures built from braided nitinol wire, a stainless-steel wire or as a laser-cut structure. The expandable structure may also function as the anchoring mechanism providing the appropriate scaffold for internally hosting a constriction that could be either of a fixed diameter or adjusted in-situ through a mechanism as disclosed herein. In some embodiments, the self-expanding structure could be short to act as proximal, medial, and distal anchors or long (potentially spanning across the anatomy) to act as a conduit of a small diameter. The conduit is a structure that creates fluid- resistance to increase the backpressure. In other embodiments, a restriction is created between inlet and outlet funnels designed and spaced to create an increase in gradient pressure while minimizing the shear stress to prevent platelet activation.

In various alternative embodiments, the orifice restriction is adjustable in-situ (e.g., in the body during the intervention) by a mechanical means located outside the restriction such as snares or other structures. Also, another embodiment increases restriction through the addition of occluding material to reduce the flow.

Monitoring and maintaining such restriction features of the disclosed embodiments can be accomplished through sensors, either passive ones or active (e.g., requiring battery). Depending on the hemodynamic feedback, the orifice restriction could be adjusted either automatically in-situ through an external stimulus or internally through a simple procedure (e.g., angioplasty balloon) or expandable in-situ to the desired diameter to achieve a beneficial hemodynamic gradient for better clinical outcome. Such hemodynamic gradient could be externally monitored through pressure and flow sensors providing such information either on-demand or constantly measured for the benefit of the physician. Acting on the results of such data, the restriction could be adjusted either in-situ or via a catheter system.

Such adjustment becomes personalized care based on the patient's physiology (either at rest or during the exercise). The restriction adjustment is achieved through a feedback loop mechanism based on the pressure gradient between the inlet and the outlet of the implant. The pressure gradient is between 1 mmHg and 10 mmHg. The pressure sensors may be located on a guidewire used to deliver the modulator device. Flow sensors on such a guidewire would be calibrated to compensate for any flow and pressure disturbance while present. Alternatively, pressure sensors could be placed at the inlet and outlet of the modulator device with a possibility to store or transmit such information or make adjustments to the restriction using an algorithm executed locally by a processor in electronics package 116, or remotely on a server communicating wirelessly with the sensors on the modulator device.

Flow modulators according to teachings of the present disclosure may be positioned at a treatment site within a patient's vasculature by delivering the modulator through the vascular system, using a suitable percutaneous delivery catheter. Suitable methods of catheterization for this purpose are known in the art. During placement procedure, a flow modulator as disclosed is maintained in its collapsed or unexpanded configuration so that its outer diameter is substantially smaller than the blood vessels through which it must pass during delivery to the treatment site. For placement in a patient's coronary sinus, a physician may insert a delivery catheter through a jugular vein or a subclavian vein, and then guide the delivery catheter into the right atrium via the superior vena cava. Another insertion point to access the coronary sinus is through a femoral vein, with the delivery catheter guided through the inferior vena cava into the right atrium. From the right atrium, the delivery catheter is navigated into coronary sinus.

In some embodiments, such as with flow modulators 920, 1120, 1220 and 1320, the sizing of entry and exit openings as well as the size of the reduced diameter portion for creating a flow reduction can be set by the physician during a pre-placement device preparation stage based on patient anatomy and clinical situation as determined at the time of the procedure. In other embodiments, such as with flow modulators 100, 300, 450 and 650, the size of the reduced diameter portion may be set after placement of the device at the treatment site and further, for embodiments such as flow modulator 100 or 650, may be dynamically altered *in vivo* in response to changing hemodynamic conditions.

Various modifications and additions can be made without departing from the spirit and scope of this disclosure. Features of each of the multiple embodiments described above may be combined with elements of other described embodiments as appropriate to provide a multiplicity of feature combinations in associated new embodiments. Furthermore, while the foregoing describes several separate embodiments, what has been described herein is merely illustrative of the application of the principles of the present disclosure. Additionally, although particular methods herein may be illustrated and/or described as being performed in a specific order, the ordering is highly variable within the ordinary skill to achieve aspects of the present disclosure. Accordingly, this description is meant to be taken only by way of example and not to limit the scope of this disclosure otherwise.

Exemplary embodiments have been disclosed above and illustrated in the accompanying drawings. It will be understood by those skilled in the art that various changes, omissions, and additions may be made to that which is specifically disclosed herein without departing from the spirit and scope of the present disclosure.

The present disclosure is further described by reference to the following numbered clauses:
Clause 1. A vascular flow modulator, comprising:
   an expandable tubular structure having first and second ends with a central portion therebetween;
   means forming an adjustable flow restriction through said central portion; and
   a thrombosis-resistant surface within the flow restriction.
Clause 2. The vascular flow modulator of clause 1, wherein said means forming an adjustable flow restriction comprises means forming an adjustable reduced diameter section of the central portion.
Clause 3. The vascular flow modulator of clause 2, wherein said means forming an adjustable reduced diameter section comprise means for adjusting the reduced diameter portion after the flow modulator is deployed within a vascular lumen of a patient.
Clause 4. The vascular flow modulator of any of clauses 1, 2 or 3, wherein:
   the expandable tubular structure comprises an outer tubular member with a first length and an inner tubular member with a second, longer length;
   the first and second tubular members are attached together at the first and second ends such that the second, longer length tubular member forms a generally hour-glass shape;
   said means forming an adjustable flow restriction comprise the first and second tubular members being extendable and compressible together in a longitudinal direction whereby a narrowing in said generally hour-glass shape changes in diameter.
Clause 5. The vascular flow modulator of any of clauses 1, 2 or 3, wherein:
   the first and second ends of the expandable tubular structure have a larger internal diameter than the internal diameter of the central portion; and
   said means forming an adjustable flow restriction comprise the central portion having a woven or braided structure that changes internal diameter in response to changes in length.
Clause 6. The vascular flow modulator of clause 4 or clause 5, further comprising a flexible tether attached to an end of the expandable tubular structure, whereby said tubular structure may be changed in length after placement in a patient's vasculature.
Clause 7. The vascular flow modulator of any of clauses 1, 2 or 3, wherein said means forming an adjustable flow restriction comprise an inflatable and deflatable balloon bearing against the central portion of the tubular structure.
Clause 8. The vascular flow modulator of clause 7, wherein:
   the expandable tubular structure comprises an inner tubular member and an outer tubular member; and
   the inflatable and deflatable balloon is disposed between said inner and outer tubular members.
Clause 9. The vascular flow modulator of clause 7 or clause 8, wherein said means forming an adjustable flow restriction further comprise:
   an electronic package including a micro-pump controlling inflation and deflation of said balloon;
   an implantable fluid reservoir configured to contain inflation fluid for said balloon; and
   fluid channel providing fluid communication between the reservoir, micro-pump and balloon.
Clause 10. The vascular flow modulator of any of clauses 1, 2 or 3, wherein said means forming an adjustable flow restriction comprises:
   an electronic package comprising a sensor controlled micro-motor; and
   an adjustable ring extending around the central portion of said tubular structure, the adjustable ring having a variable diameter driven by the micro-motor.
Clause 11. The vascular flow modulator of clause 9 or clause 10, wherein the electronics package further comprises a pressure sensor communicating with the micro-motor or micro-pump, whereby the diameter of the narrowed central portion of said tubular structure is varied in response to changes in vascular pressure.
Clause 12. The vascular flow modulator of any of clauses 1, 2 or 3, wherein said means forming an adjustable flow restriction comprises an adjustable ring surrounding the central portion of the expandable tubular structure and a flexible tether manipulable from outside the patient's vasculature cooperating with the adjustable ring to alter the adjustable ring diameter.
Clause 13. The vascular flow modulator of any of clauses 1, 2 or 3, , wherein the expandable tubular structure comprises an invaginated mesh tube.
Clause 14. The vascular flow modulator of clause 13, wherein said means forming an adjustable flow restriction comprise a first end of the invaginated tube inserted into a second end of the invaginated tube, the second end of the invaginated tube having a raised inner annular ridge, whereby the diameter of the flow restriction is adjustable based on the distance of insertion of the first end into the second end.
Clause 15. The vascular flow modulator of clause 13, wherein said means forming an adjustable flow restriction comprise an annular collar with a predetermined internal diameter joining opposite ends of the invaginated mesh tube.
Clause 16. The vascular flow modulator of clause 15, wherein said means forming an adjustable flow restriction comprise a plurality of said annular collars, each with a different internal diameter, whereby selection of different annular collars provides for adjustment of the internal diameter of the adjustable flow restriction.
Clause 17. The vascular flow modulator of clause 1 or clause 2, wherein said means forming an adjustable flow restriction comprise a first sleeve surrounding the central portion of the expandable tubular structure and constraining the tubular structure in the central portion at a pre-selected reduced diameter.
Clause 18. The vascular flow modulator of clause 17, wherein said first sleeve has a length corresponding to a length of the constrained reduced diameter portion of said central portion.
Clause 19. The vascular flow modulator of clause 17 or clause 18, wherein:
   said first sleeve is selected from a plurality of first sleeves having different internal diameters, whereby a change in sleeve provides for adjustment of said reduced diameter portion; and
   said first sleave is configured to be cut to a desired length to set the length of the constrained reduced diameter portion of said central portion.
Clause 20. The vascular flow modulator of any of clauses 17, 18, or 19 further comprising a second, outer sleeve surrounding the expandable tubular structure and the first sleeve, said second sleeve having at least an outer facing surface comprised of a tissue in-growth promoting coating or material.
Clause 21. The vascular flow modulator of any of clauses 17, 18, 19 or 20 , wherein at least one of said first sleeve and said second sleeve is formed of a sheet of self-adhering material rolled onto itself, whereby an internal diameter of the first or second sleeve may be set by a physician before or during a procedure to place said vascular flow restrictor.
Clause 22. The vascular flow modulator of any of clauses 17, 18, 19, 20 or 21 , wherein the first sleeve has at least an inner surface comprised of a thrombosis-resistant material or coating.
Clause 23. The vascular flow modulator of clause 1, wherein:
   the central portion comprises a plurality of smaller tubes, each of said smaller tubes having a diameter smaller than the expandable tubular structure; and
   said means forming an adjustable flow restriction comprises said first and second ends being rotatable relative to each other whereby the smaller tubes are twisted to create a flow path with greater or lesser tortuosity.
Clause 24. A vascular flow modulator kit, comprising:
   an open cell expandable tubular structure having first and second ends with a narrowed central portion therebetween;
   a first sleeve material having at least an inner thrombosis-resistant surface and formable into a plurality of first sleeves of different lengths and inner diameters, said first sleeves when placed around the narrowed central portion of the expandable tubular structure constrain said narrowed central portion at a selected inner diameter and length with an inward facing thrombosis-resistant surface; and
   a second sleeve material having at least an outer tissue in-growth promoting surface and formable into a plurality of second sleeves of different lengths and diameters, said second sleeves configured to surround at least the first sleeve to provide an outward facing tissue in-growth promoting surface.
Clause 25. The vascular flow modulator kit of clause 24, wherein at least one of said first sleeve material and said second sleeve material is provided as preformed sleeves.
Clause 26. The vascular flow modulator kit of clause 24 or clause 25, wherein at least one of said first sleeve material and said second sleeve material is provided as a sheet of self-adhering material, whereby an internal diameter of the first or second sleeve may be set by a physician before or during a procedure to place a vascular flow restrictor formed with said kit.
Clause 27. The vascular flow modulator or modulator kit of any of clauses 1-2426, wherein the expandable tubular structure is self-expanding.
Clause 28. The vascular flow modulator or modulator kit of any of clauses 1-27, wherein the expandable tubular structure comprises a braided or wire mesh structure.
Clause 29. The vascular flow modulator or modulator kit of any of clauses 1-27, wherein the expandable tubular structure comprises a laser-cut tubular structure.
Clause 30. A method of modulating vascular flow, comprising:
   preparing a vascular flow modulator for placement in a patient;
   delivering the vascular flow modulator through the patient's vasculature to a treatment site;
   placing the vascular flow modulator within a vascular lumen at the treatment site; and
   adjusting a narrowed flow reducing portion of the vascular flow modulator based on specific patient clinical need.
Clause 31. The method of clause 30, wherein said adjusting the narrowed flow reducing portion comprises configuring the vascular flow modulator during said preparing.
Clause 32. The method of clause 30, wherein said adjusting the narrowed flow reducing portion comprises changing an internal diameter of the narrowed flow reducing portion after placing the vascular flow modulator in the vascular lumen.
Clause 33. The method of clause 31 or clause 32, wherein said adjusting the narrowed flow reducing portion comprises placing an adjustable ring around a central portion of the vascular flow modulator.
Clause 34. The method of clause 33, wherein the adjustable ring is adjusted in diameter *in vivo* after placement of the vascular flow modulator in a vascular lumen.
Clause 35. The method of clause 31 or clause 32, wherein said adjusting the narrowed flow reducing portion comprises joining a first end of an invaginated tube structure with a second end of said tube structure.
Clause 36. The method of clause 31 or clause 32, wherein said adjusting the narrowed flow reducing portion comprises increasing the length of the vascular flow modulator.
Clause 37. The method of clause 31 or clause 32, wherein said adjusting the narrowed flow reducing portion comprises decreasing the length of the vascular flow modulator.
Clause 38. The method of clause 31 or clause 32, wherein said adjusting the narrowed flow reducing portion comprises inflating or deflating a balloon bearing on a central portion of the vascular flow modulator.
Clause 39. The method of clause 38, wherein inflating or deflating comprises inflating or deflating the balloon *in vivo* automatedly in response to changes in a measured hemodynamic parameter.
Clause 40. The method of clause 31 or clause 32, wherein said adjusting the narrowed flow reducing portion comprises manipulating a flexible tether attached to the flow modulator.
Clause 41. The method of clause 31 or clause 32, wherein said adjusting the narrowed flow reducing portion comprises rotating one end of the flow modulator relative to an opposite end of the flow modulator.
Clause 42. The method of clause 31 or clause 32, wherein said adjusting the narrowed flow reducing portion comprises placing a sleeve with a selected diameter around a central portion of the flow modulator.
Clause 43. The method of clause 30, wherein the vascular flow modulator comprises a vascular flow modulator according to any of clauses 1-29.

## Claims

1. A vascular flow modulator, comprising:
an expandable tubular structure (920, 1120, 1220, 1320) having first and second ends (926, 928, 1126, 1128, 1226, 1228, 1326, 1328) with a central portion (924, 1124) therebetween;
means forming an adjustable flow restriction through said central portion (924, 1124); and
a thrombosis-resistant surface within the flow restriction,
wherein said means forming an adjustable flow restriction comprises a flexible tether and, alternatively, the central portion having a woven or braided structure that changes internal diameter in response to changes in length wherein the flexible tether is attached to an end of the expandable tubular structure, whereby said tubular structure may be changed in length after placement in a patient's vasculature, or an adjustable ring surrounding the central portion of the expandable tubular structure wherein the flexible tether manipulable from outside the patient's vasculature cooperating with the adjustable ring to alter the adjustable ring diameter.

2. The vascular flow modulator of claim 1, wherein said means forming an adjustable flow restriction is operable to form an adjustable reduced diameter section of the central portion.

3. The vascular flow modulator of any of claims 1 or 2, wherein the first and second ends of the expandable tubular structure have a larger internal diameter than the internal diameter of the central portion.

4. The vascular flow modulator of any of claims 1, 2 or 3, wherein the flexible tether and adjustable ring are configured to change the diameter of the adjustable ring in response to pulling or releasing the flexible tether from outside the patient's vasculature.

5. The vascular flow modulator of claim 4, further comprising an anchor wire or one or both ends of the tubular structure.

6. The vascular flow modulator of any of claims 1-5, further comprising:
an electronic package comprising a sensor controlled micro-motor, wherein the adjustable ring diameter is variable by the micro-motor; and
a pressure sensor communicating with the micro-motor, whereby the diameter of the narrowed central portion of said tubular structure is varied in response to changes in vascular pressure.
